(19) Europäisches Patentamt
European Patent Office
Office européen des brevets

(11) **EP 3 477 903 A1**

(12) **EUROPÄISCHE PATENTANMELDUNG**

(43) Veröffentlichungstag:
**01.05.2019 Patentblatt 2019/18**

(51) Int Cl.:
***H04L 29/06*** *(2006.01)*　　　***G06F 21/62*** *(2013.01)*
***G16H 10/60*** *(2018.01)*

(21) Anmeldenummer: **17199022.9**

(22) Anmeldetag: **27.10.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Siemens Healthcare GmbH**
**91052 Erlangen (DE)**

(72) Erfinder:
- **Bertsch, Rüdiger**
**91052 Erlangen (DE)**
- **Tintemann, Falk**
**91336 Heroldsbach (DE)**

(54) **ÜBERTRAGEN EINES VERTRAULICHEN MEDIZINISCHEN DATENSATZES, INSBESONDERE ZUR FERNBEFUNDUNG**

(57)　Die Erfindung basiert darauf, dass ein Transaktionsdatensatz umfassend einen verschlüsselten Datensatz mittels einer Datenquelle (10) bestimmt wird, wobei der verschlüsselte Datensatz auf einer Verschlüsselung des vertraulichen Datensatzes basiert. Weiterhin wird der Transaktionsdatensatz von der Datenquelle (10) zu einer zentralen Distributionseinheit (30) übertragen. Weiterhin wird durch die zentrale Distributionseinheit (30) eine erste Verarbeitungsbedingung empfangen, wobei die erste Verarbeitungsbedingung eine Bedingung für die Verarbeitbarkeit des vertraulichen Datensatzes durch eine von mehreren Verarbeitungseinheiten (50, 60, 70) ist. Weiterhin wird mittels der zentralen Distributionseinheit (30) eine empfangende Verarbeitungseinheit (50) aus den Verarbeitungseinheiten (50, 60, 70) ausgewählt, wobei die empfangende Verarbeitungseinheit (50) die erste Verarbeitungsbedingung erfüllt. Weiterhin wird der Transaktionsdatensatz von der zentralen Distributionseinheit (30) zu der empfangenden Verarbeitungseinheit (50) übertragen. Weiterhin wird der mittels der empfangenden Verarbeitungseinheit (50) der vertrauliche Datensatz basierend auf einer Entschlüsselung des verschlüsselten Datensatzes bestimmt, insbesondere des im Transaktionsdatensatz enthaltenen verschlüsselten Datensatzes.

FIG 1

EP 3 477 903 A1

**Beschreibung**

Aufgrund von zunehmender Spezialisierung von Fachpersonen ist es nicht möglich oder zumindest nicht wirtschaftlich, dass alle zur Bearbeitung von Aufträgen notwendige Fachpersonen räumlich an einem Ort zusammenarbeiten. Beispielsweise ist es wirtschaftlich nicht darstellbar, dass in einem eher kleinen Krankenhaus alle radiologischen Fachrichtungen vertreten sind. In diesen Situationen ist virtuelle Zusammenarbeit zwischen räumlich voneinander entfernten Institutionen bzw. räumlich voneinander entfernten Fachpersonen nötig und möglich. Im dargestellten medizinischen Umfeld steht beispielsweise die Methode der Fernbefundung zur Verfügung, wobei ein fachlich besonders qualifizierter Radiologe die fachgerechte Interpretation der radiologischen Bilder und klinischen Informationen vornimmt und dabei räumlich von der bildgebenden medizinischen Modalität entfernt ist.

Für eine solche virtuelle Zusammenarbeit ist es notwendig, vertrauliche Datensätze derart teilnehmenden Fachpersonen bzw. teilnehmenden Institutionen zu verschicken, dass kein unbefugter Dritter Zugang zu den vertraulichen Datensätzen erhält. Dies gilt insbesondere im medizinischen Umfeld, da hier vertrauliche Datensätze insbesondere personenbezogene Daten von Patienten enthalten können, beispielsweise Ergebnisse von medizinischen Bildgebungsverfahren oder Laborergebnisse. Insbesondere bei der radiologischen Fernbefundung müssen sensible und patientenbezogene Bilddaten von einer medizinischen Einrichtung, welche bildgebende medizinische Vorrichtungen betreibt (beispielsweise ein Krankenhaus oder eine Radiologiepraxis), zur Befundung an einen räumlich von der ersten Einrichtung getrennten Arbeitsplatz (beispielsweise einen Telearbeitsplatz eines Radiologen) geschickt werden, ohne dass ein unbefugter Dritter Einsicht in diese Bilddaten nehmen kann. Weiterhin ist es notwendig, auf dem Übertragungsweg die Integrität der vertraulichen Datensätze sicherzustellen, d.h. sie gegen eine mögliche Manipulation zu schützen.

Hierfür ist es bekannt, vertrauliche Datensätze in einer verschlüsselten Form unter Verwendung von asymmetrischer Kryptographie auszutauschen. Durch die asymmetrische Kryptographie, insbesondere durch die Verwendung von öffentlichen und geheimen Schlüsseln), kann hierbei das Problem des Schlüsselaustausches umgangen werden, und gleichzeitig die Urheberschaft an dem vertraulichen Datensatz durch eine elektronische Signatur verifiziert werden. Hierbei muss aber der Empfänger des vertraulichen Datensatzes dem Sender des vertraulichen Datensatzes vor dem Versenden der verschlüsselten Form des vertraulichen Datensatzes bekannt sein, da nur so der öffentliche Schlüssel des Empfängers zur Verschlüsselung herangezogen werden kann. Insbesondere können die vertraulichen Datensätze daher nicht an mehrere (potentielle) Empfänger geschickt werden, ohne die vertraulichen Datensätze jeweils separat mit dem jeweiligen öffentlichen Schlüssel zu verschlüsseln.

Durch das Versenden der vertraulichen Datensätze nur an einen vorab bestimmten Empfänger können Ineffizienzen auftreten. Beispielsweise kann der designierte Empfänger für einen längeren Zeitraum nicht verfügbar sein bzw. bereits mit der Abarbeitung von anderen vertraulichen Datensätzen beschäftigt sein.

Es ist bekannt, für die effiziente Verteilung von Datensätzen zentrale Systeme zu verwenden, welche Datensätze nach vorbestimmten Kriterien an Datenempfänger zur Weiterverarbeitung sendet. Um einen sicheren Transport der Datensätze zwischen dem zentralen System und den Datenempfängern zu ermöglichen, beispielsweise mittels eines asymmetrischen Kryptographiesystems, muss das zentrale System aber Zugriff auf die unverschlüsselten Daten haben.

Es ist daher die Aufgabe der vorliegenden Erfindung, eine Methode bereitzustellen, welche eine effiziente und sichere Verteilung von vertraulichen Datensätzen zwischen Datenquellen und Datenverarbeitern über ein zentrales System ermöglicht, insbesondere für die Verteilung von medizinischen Datensätzen zur Fernbefundung.

Die Aufgabe wird gelöst durch ein Verfahren zum Übertragen eines vertraulichen Datensatzes nach Anspruch 1, durch ein Übertragungssystem nach Anspruch 12, durch ein Computerprogrammprodukt nach Anspruch 14 sowie durch ein computerlesbares Speichermedium nach Anspruch 15.

Nachstehend wird die erfindungsgemäße Lösung der Aufgabe sowohl in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Die Erfindung basiert darauf, dass ein Transaktionsdatensatz umfassend einen verschlüsselten Datensatz mittels einer Datenquelle bestimmt wird, wobei der verschlüsselte Datensatz auf einer Verschlüsselung des vertraulichen Datensatzes basiert. Weiterhin wird der Transaktionsdatensatz von der Datenquelle zu einer zentralen Distributionseinheit übertragen. Weiterhin wird durch die zentrale Distributionseinheit eine erste Verarbeitungsbedingung empfangen, wobei die erste Verarbeitungsbedingung eine Bedingung für die Verarbeitbarkeit des vertraulichen Datensatzes durch eine von mehreren Verarbeitungseinheiten ist. Weiterhin wird mittels der zentralen Distributionseinheit eine empfangende Verarbeitungseinheit aus den Verarbeitungseinheiten ausgewählt, wobei die empfangende Verarbeitungseinheit die erste Verarbeitungsbedingung erfüllt. Weiterhin wird der Transaktionsdatensatz von der zentralen Distributionseinheit zu der empfangenden Verarbeitungseinheit übertragen. Weiterhin wird der mittels der empfangenden Verarbeitungseinheit der vertrauliche Datensatz basierend auf einer Entschlüsselung des verschlüsselten Datensatzes bestimmt, insbesondere des im Transaktionsdatensatz enthaltenen verschlüsselten Datensatzes.

Die Erfinder haben erkannt, dass es durch diese Methode möglich ist, einen vertraulichen Datensatz flexibel zu einer empfangenden von mehreren Verarbeitungseinheiten zu schicken, ohne dass der vertrauliche Datensatz von der zentralen Distributionseinheit eingesehen werden kann. Durch das Auswähle basierend auf der ersten Verarbeitungsbedingung kann sichergestellt werden, dass der verschlüsselte vertrauliche Datensatz nur an geeignete oder zugelassene Verarbeitungseinheiten geschickt wird.

Das erste Bestimmen des Transaktionsdatensatzes wird insbesondere mittels einer Recheneinheit der Datenquelle ausgeführt. Das erste Übertragen des Transaktionsdatensatzes von der Datenquelle zu einer zentralen Distributionseinheit wird insbesondere von einer Schnittstelle der Datenquelle und von einer Schnittstelle der zentralen Distributionseinheit ausgeführt, das erste Übertragen erfolgt insbesondere mittels eines ersten Netzwerkes. Das erste Empfangen der ersten Verarbeitungsbedingung wird insbesondere durch die Schnittstelle der zentralen Distributionseinheit ausgeführt. Das Auswählen einer empfangenden Verarbeitungseinheit wird insbesondere mittels einer Recheneinheit der zentralen Distributionseinheit durchgeführt. Das zweite Übertragen des Transaktionsdatensatzes von der zentralen Distributionseinheit zu der empfangenden Verarbeitungseinheit wird insbesondere mittels der Schnittstelle der zentralen Distributionseinheit und mittels einer Schnittstelle der empfangenden Verarbeitungseinheit durchgeführt, das zweite Übertragen erfolgt insbesondere über ein zweites Netzwerk. Hierbei kann das zweite Netzwerk insbesondere auch identisch mit dem ersten Netzwerk sein. Das zweite Bestimmen des vertraulichen Datensatzes wird insbesondere durch eine Recheneinheit der empfangenden Verarbeitungseinheit ausgeführt.

Nach einem weiteren Aspekt der Erfindung ist der vertrauliche Datensatz ein medizinischer Datensatz, insbesondere ein medizinischer Bilddatensatz. Das Verfahren umfasst weiterhin das Bereitstellen des vertraulichen Datensatzes zur medizinischen Befundung mittels der empfangenden Verarbeitungseinheit, das zweite Empfangen einer Befundungsinformation mittels der empfangenden Verarbeitungseinheit, und das dritte Übertragen der Befundungsinformation von der empfangenden Verarbeitungseinheit zur Datenquelle. Insbesondere kann hierbei die Befundungsinformation auf dem medizinischen Datensatz basieren. Insbesondere kann die Befundungsinformation mittels der empfangenden Verarbeitungseinheit verschlüsselt werden, die Befundungsinformation verschlüsselt von der empfangenden Verarbeitungseinheit zur Datenquelle übertragen werden, und von der Datenquelle entschlüsselt werden. Das Bereitstellen des vertraulichen Datensatzes sowie das zweite Empfangen einer Befundungsinformation kann insbesondere mittels der Schnittstelle der empfangenden Verarbeitungseinheit ausgeführt werden. Das dritte Übertragen der Befindungsinformation kann insbesondere mittels der Schnittstelle der empfangenden Verarbeitungseinheit und mittels der Schnittstelle der Datenquelle ausgeführt werden. Die Erfinder haben erkannt, dass basierend auf diesem Aspekt der Erfindung eine Befundung des vertraulichen, hier medizinischen Datensatzes besonders effizient erfolgen kann, beispielsweise innerhalb eines Fernbefundungssystems. Insbesondere kann dann auch die Befundungsinformation wieder zur Datenquelle übertragen werden, ohne von einem unbefugten Dritten einsehbar zu sein.

Nach einem weiteren möglichen Aspekt der Erfindung erfolgt das dritte Übertragen der Befundungsinformation von der empfangenden Verarbeitungseinheit zur Datenquelle dadurch, dass die Befundungsinformation von der empfangenden Verarbeitungseinheit zur zentralen Distributionseinheit übertragen wird, und dass die Befundungsinformation anschließend von der zentralen Distributionseinheit zur Datenquelle übertragen wird.

Nach einem weiteren Aspekt der Erfindung ist die Verschlüsselung des vertraulichen Datensatzes eine symmetrische Verschlüsselung basierend auf einem Datenschlüssel, wobei die Entschlüsselung des verschlüsselten Datensatzes eine symmetrische Entschlüsselung basierend auf dem Datenschlüssel ist. Weiterhin umfasst dieser Aspekt des Verfahrens das verschlüsselte Übertragen des Datenschlüssels von der Datenquelle zu der empfangenden Verarbeitungseinheit. Hierbei ist das symmetrische Entschlüsseln eine inverse Operation des symmetrischen Verschlüsselns. Insbesondere kann das Übertragen des Datenschlüssels auch über die zentrale Distributionseinheit erfolgen. Insbesondere kann das verschlüsselte Übertragen auf einer Anforderung der zentralen Distributionseinheit als Reaktion auf das Auswählen der empfangenden Verarbeitungseinheit erfolgen. Die Erfinder haben erkannt, dass durch diesen Aspekt des Verfahrens der Datenschlüssel flexibel und sicher an die empfangende Verarbeitungseinheit übermittelt werden kann.

Nach einem weiteren Aspekt der Erfindung wird der Datenschlüssel nur für die Verschlüsselung von genau einem vertraulichen Datensatz verwendet. Die Erfinder haben erkannt, dass durch eine nur einmalige Verwendung des Datenschlüssels der vertrauliche Datensatz besser geschützt ist, da die Entschlüsselung erst nach der Übertragung des einmaligen Datenschlüssels erfolgen kann und der Zeitpunkt der Entschlüsselung daher unabhängig von dem Zeitpunkt des Übertragens des Transaktionsdatensatzes gewählt und gesteuert werden kann. Bei einer mehrmaligen Verwendung eines Datenschlüssels könnte die empfangende Verarbeitungseinheit einen Transaktionsdatensatz direkt nach dessen Empfang entschlüsseln.

Nach einem weiteren Aspekt der Erfindung basiert das verschlüsselte Übertragen des Datenschlüssels auf einem asymmetrischen Kryptographiesystem. Insbesondere ist hierbei jeder der Verarbeitungseinheiten ein Schlüsselpaar umfassend einen geheimen Schlüssel und einen öffentlichen Schlüssel zugeordnet, wobei der geheime Schlüssel nur durch die jeweilige Verarbeitungseinheit zugänglich ist. Zur Übertragung wird der Datenschlüssel dann insbesondere mit dem öffentlichen Schlüssel der empfangenden Verarbeitungseinheit durch die Datenquelle verschlüsselt und nach dem Empfangen durch die empfangende Verarbeitungseinheit mit dem geheimen Schlüssel entschlüsselt. Die Erfinder haben erkannt, dass durch ein asymmetrisches Kryptographiesystem das Problem des Schlüsselaustausches umgangen werden kann, und das Verfahren also besonders einfach und sicher durchgeführt werden kann.

Nach einem weiteren möglichen Aspekt der Erfindung ist der Transaktionsdatensatz oder der vertrauliche Datensatz basierend auf einem Schlüsselpaar der Datenquelle signiert. Das Schlüsselpaar der Datenquelle umfasst insbesondere einen öffentlichen und einen geheimen Schlüssel. Das Signieren des Transaktionsdatensatzes basiert insbesondere auf dem geheimen Schlüssel der Datenquelle, die Signatur kann insbesondere mittels des öffentlichen Schlüssels der Datenquelle verifiziert werden. Der Transaktionsdatensatz kann insbesondere die Signatur umfassen, die Signatur kann aber auch separat vom Transaktionsdatensatz übertragen werden. Da eine Signatur keine vertraulichen Daten umfasst, kann die Signatur auch insbesondere auf der zentralen Distributionseinheit gespeichert werden. Die Erfinder haben erkannt, dass durch eine Signatur die Urheberschaft des vertraulichen Datensatzes verifiziert werden kann, und gleichzeitig der vertrauliche Datensatz gegen Manipulationen geschützt werden kann.

Nach einem weiteren möglichen Aspekt der Erfindung basiert das verschlüsselte Übertragen des Datenschlüssels auf einem quantensicheren Kryptographiesystem. Ein quantensicheres Kryptographiesystem kann insbesondere auf Quanteneffekten basieren, oder insbesondere Verschlüsselungstechniken umfassen, deren Entschlüsselung auch für eine auf Quanteneffekten basierende Recheneinheit in der NP Komplexitätsklasse liegt. Beispiele sind der Quantenschlüsselaustausch, oder klassische Algorithmen basierend auf mathematischen Gittern (z.B. Ring-LWE, eine Abkürzung für "ring learning with errors" oder NTRUEncrypt), basierend auf multivariaten Polynomen (z.B. das Unbalanced-Oil-and-Vinegar-Verfahren) oder basierend auf fehlerkorrigierenden Codes (z.B. das McEliece-Kryptosystem). Die Erfinder haben erkannt, dass durch ein quantensicheres Kryptographiesystem das Übertragungsverfahren auch sicher gegenüber zukünftigen böswilligen Entschlüsselungsversuchen ist.

Nach einem weiteren Aspekt der Erfindung wird das verschlüsselte Übertragen des Datenschlüssels von der empfangenden Verarbeitungseinheit initiiert, wenn mittels der empfangenden Verarbeitungseinheit erstmals ein Zugriff auf den vertraulichen Datensatz erfolgt. Die Erfinder haben erkannt, dass es durch diese späte Übertragung des Datenschlüssels noch möglich ist, die Übermittlung des verschlüsselten Datensatzes im Zeitraum zwischen dem Übertragen des Transaktionsdatensatzes von der zentralen Distributionseinheit zur empfangenden Datenquelle und dem erstmaligen Zugriff auf den Transaktionsdatensatz bzw. den darin enthaltenen vertraulichen Datensatz rückgängig zu machen (indem der zugehörige Datenschlüssel nie übertragen wird).

Nach einem weiteren Aspekt der Erfindung basiert die erste Verarbeitungsbedingung darauf, ob eine vertragliche Regelung zwischen einem ersten Betreiber der Datenquelle und einem zweiten Betreiber einer der Verarbeitungseinheiten besteht. Insbesondere kann hierbei beim ersten Empfang der ersten Verarbeitungsbedingung für die Datenquelle eine Liste mit zweiten Betreibern bzw. den Verarbeitungseinheiten der zweiten Betreiber empfangen werden, wobei zwischen den zweiten Betreibern und den ersten Betreibern eine vertragliche Regelung besteht. Insbesondere kann hierbei auch beim ersten Empfangen der ersten Verarbeitungsquelle eine solche Liste für alle möglichen Datenquellen empfangen werden, insbesondere in Form einer Matrix oder Tabelle, welche die Existenz einer vertraglichen Regelung enthält. Die vertragliche Regelung regelt hierbei insbesondere, dass eine Verarbeitungseinheit vertrauliche Datensätze der Datenquelle verarbeiten kann. Die Erfinder haben erkannt, dass mit diesem Aspekt der Erfindung sichergestellt werden kann, dass vertrauliche Datensätze nur von zur Verarbeitung zugelassenen Verarbeitungseinheiten verarbeitet werden.

Nach einem weiteren Aspekt der Erfindung wird beim ersten Empfangen weiterhin eine Verarbeitungsinformation empfangen, wobei die Verarbeitungsinformation wenigstens einen der folgenden Parameter betrifft:

- Zeitraum, der zur Befundung des vertraulichen Datensatzes zur Verfügung steht,
- Notwendige Qualifikation zur Befundung des vertraulichen Datensatzes,
- Preisrahmen für eine Befundung des vertraulichen Datensatzes.

Die Erfinder haben erkannt, dass es basierend auf einer derartigen Verarbeitungsinformation die vertraulichen Datensätze besonders effizient auf die Verarbeitungseinheiten verteilt werden können.

Nach einem weiteren Aspekt der Erfindung umfasst die Verarbeitungsinformation eine zweite Verarbeitungsbedingung, wobei die empfangende Verarbeitungseinheit die zweite Verarbeitungsbedingung erfüllt. Die Erfinder haben erkannt, dass basierend auf einer zweiten Verarbeitungsbedingung eine besonders effiziente Auswahl der empfangenden Verarbeitungseinheit möglich ist.

Nach einem weiteren Aspekt der Erfindung wird die empfangende Verarbeitungseinheit zufällig aus einer Untermenge der Verarbeitungseinheiten ausgewählt, wobei jede der Verarbeitungseinheiten der Untermenge die erste Verarbeitungsbedingung und die zweite Verarbeitungsbedingung erfüllt. Die Erfinder haben erkannt, dass basierend auf einer zufälligen Auswahl sichergestellt werden kann, dass die Befundung des vertraulichen Datensatzes möglichst objektiv erfolgt und insbesondere für die Befundung nachteilige wirtschaftliche Anreize ausgeschlossen werden können.

Nach einem weiteren Aspekt der Erfindung basiert das Auswählen der empfangenden Verarbeitungseinheit aus den Verarbeitungseinheiten auf der Auswertung einer Kostenfunktion für die Verarbeitungseinheiten, wobei die Kostenfunktion auf der Verarbeitungsinformation basiert. Insbesondere wird hierbei die Verarbeitungsinformation mit entsprechenden Parametern verglichen, die von den Verarbeitungseinheiten zur Verfügung gestellt werden. Die Erfinder haben erkannt, dass basierend auf einer Kostenfunktion eine besonders effiziente und bedarfsgerechte Verteilung von vertraulichen Datensätzen auf die Verarbeitungseinheiten möglich ist.

Die Erfindung kann auch ein Verfahren zum Verteilen eines vertraulichen Datensatzes betreffen, insbesondere ausgeführt mittels einer zentralen Distributionseinheit, welches die folgenden Verfahrensschritte umfasst:

- Empfangen eines Transaktionsdatensatzes mittels einer Schnittstelle,
  wobei der Transaktionsdatensatz einen verschlüsselten Datensatz umfasst,
  wobei der verschlüsselte Datensatz auf einer Verschlüsselung des vertraulichen Datensatzes basiert,
- Empfangen einer ersten Verarbeitungsbedingung mittels der Schnittstelle,
- Auswählen einer empfangenden Verarbeitungseinheit aus den Verarbeitungseinheiten mittels einer Recheneinheit,
  wobei die empfangende Verarbeitungseinheit die erste Verarbeitungsbedingung erfüllt,
- Senden des Transaktionsdatensatzes mittels der Schnittstelle zu der empfangenden Verarbeitungseinheit.

Vorteilhafte Weiterbildungen und Ausführungsformen des Verfahrens zum Übertragen eines Datensatzes, insbesondere der Verfahrensschritte des Verfahrens zum Übertragen eines Datensatzes, können einzeln oder in einer beliebigen Kombination auch auf das Verfahren zum Verteilen eines vertraulichen Datensatzes, insbesondere auf die einzelnen Verfahrensschritte des Verfahrens zum Verteilen eines vertraulichen Datensatzes angewendet und übertragen werden.

Die Erfindung betrifft weiterhin ein Übertragungssystem zum Übertragen eines vertraulichen Datensatzes, umfassend die folgenden Einheiten:

- Datenquelle, ausgebildet zum ersten Bestimmen eines Transaktionsdatensatzes umfassend einen verschlüsselten Datensatz,
  wobei der verschlüsselte Datensatz auf einer Verschlüsselung des vertraulichen Datensatzes basiert,
  weiterhin ausgebildet zum ersten Übertragen des Transaktionsdatensatzes zu einer zentralen Distributionseinheit,
- zentrale Distributionseinheit, ausgebildet zum ersten Empfangen einer ersten Verarbeitungsbedingung, wobei die erste Verarbeitungsbedingung eine Bedingung für die Verarbeitbarkeit des vertraulichen Datensatzes durch eine von mehreren Verarbeitungseinheiten ist,
  weiterhin ausgebildet zum Auswählen einer empfangenden Verarbeitungseinheit aus den Verarbeitungseinheiten,
  wobei die empfangende Verarbeitungseinheit die erste Verarbeitungsbedingung erfüllt,
  weiterhin ausgebildet zum zweiten Übertragen des Transaktionsdatensatzes zu der empfangenden Verarbeitungseinheit,
- empfangende Verarbeitungseinheit, ausgebildet zum zweiten Bestimmen des vertraulichen Datensatzes basierend auf einer Entschlüsselung des verschlüsselten Datensatzes.

Ein solches Übertragungssystem kann insbesondere dazu ausgebildet sein die zuvor beschriebenen erfindungsgemäßen Verfahren und ihre Aspekte auszuführen. Das Übertragungssystem ist dazu ausgebildet diese Verfahren und ihre Aspekte auszuführen, indem die Datenquelle, die zentrale Distributionseinheit und die empfangende Verarbeitungseinheit ausgebildet sind, die entsprechenden Verfahrensschritte auszuführen. Die Datenquelle ist insbesondere dazu ausgebildet, einen entsprechenden Verfahrensschritt auszuführen, indem eine Schnittstelle und eine Recheneinheit der Datenquelle dazu ausgebildet sind, den entsprechenden Verfahrensschritt auszuführen. Die zentrale Distributionseinheit ist insbesondere dazu ausgebildet, einen entsprechenden Verfahrensschritt auszuführen, indem eine Schnittstelle und eine Recheneinheit der zentralen Distributionseinheit dazu ausgebildet sind, den entsprechenden Verfahrensschritt auszuführen. Die empfangende Verarbeitungseinheit ist insbesondere dazu ausgebildet, einen entsprechenden Verfahrensschritt auszuführen, indem eine Schnittstelle und eine Recheneinheit der empfangenden Verarbeitungseinheit dazu ausgebildet sind, den entsprechenden Verfahrensschritt auszuführen.

Die Erfindung betrifft auch ein Computerprogrammprodukt mit einem Computerprogramm sowie ein computerlesbares Medium. Eine weitgehend softwaremäßige Realisierung hat den Vorteil, dass auch schon bisher verwendete Übertragungssystem auf einfache Weise durch ein Software-Update nachgerüstet werden können, um auf die erfindungsgemäße Weise zu arbeiten. Ein solches Computerprogrammprodukt kann neben dem Computerprogramm gegebenenfalls zusätzliche Bestandteile wie z. B. eine Dokumentation und/oder zusätzliche Komponenten, sowie Hardware-Komponenten, wie z.B. Hardware-Schlüssel (Dongles etc.) zur Nutzung der Software, umfassen.

Hierbei ist der Speicher des Übertragungssystems als Gesamtheit von Speichereinheiten der Datenquelle, der zentralen Distributionseinheit und der empfangenden Verarbeitungseinheit zu verstehen. Ein Computerprogramm ist insbesondere im Speicher eines Übertragungssystems gespeichert, wenn es im Ganzen oder in Teilen in einem oder in mehreren Speichereinheiten der Datenquelle, der zentralen Distributionseinheit und der empfangenden Verarbeitungseinheit gespeichert ist. Weiterhin ist unter einer Ausführung von Programmabschnitten durch das Übertragungssystem zu verstehen, dass die Programmabschnitte von der Datenquelle, der zentralen Distributionseinheit und/oder der empfangenden Verarbeitungseinheit ausgeführt werden, insbesondere von einer Recheneinheit der Datenquelle, einer Recheneinheit der zentralen Distributionseinheit und/oder einer Recheneinheit der empfangenden Verarbeitungseinheit.

Die Erfindung kann auch eine zentrale Distributionseinheit betreffen, umfassend die folgenden Einheiten:

- Schnittstelle, ausgebildet zum Empfangen eines Transaktionsdatensatzes, wobei der Transaktionsdatensatz einen verschlüsselten Datensatz umfasst, wobei der verschlüsselte Datensatz auf einer Verschlüsselung des vertraulichen Datensatzes basiert,
weiterhin ausgebildet zum Empfangen einer ersten Verarbeitungsbedingung mittels der Schnittstelle,
weiterhin ausgebildet zum Senden des Transaktionsdatensatzes zu der empfangenden Verarbeitungseinheit,
- Recheneinheit, ausgebildet zum Auswählen einer empfangenden Verarbeitungseinheit aus den Verarbeitungseinheiten, wobei die empfangende Verarbeitungseinheit die erste Verarbeitungsbedingung erfüllt.

Die Erfindung kann auch eine Datenquelle und/oder eine empfangende Datenverarbeitungseinheit betreffen, wobei die Datenquelle dazu ausgebildet ist, im Rahmen des erfindungsgemäßen Verfahrens zum Übertragen eines vertraulichen Datensatzes mit einer zentralen Distributionseinheit und/oder einer empfangenden Verarbeitungseinheit zu interagieren, und/oder wobei die empfangende Datenverarbeitungseinheit dazu ausgebildet ist, im Rahmen des erfindungsgemäßen Verfahrens oder einer seiner Aspekte mit einer zentralen Distributionseinheit und/oder einer empfangenden Verarbeitungseinheit zu interagieren.

Die Erfindung kann auch ein Computerprogrammprodukt mit einem Computerprogramm betreffen, welches direkt in einen Speicher einer zentralen Distributionseinheit ladbar ist, mit Programmabschnitten, um alle Schritte des Verfahrens zum Verteilen eines vertraulichen Datensatzes auszuführen, wenn die Programmabschnitte von der zentralen Distributionseinheit ausgeführt werden. Die Erfindung kann auch ein computerlesbares Speichermedium betreffen, auf welchem von einer zentralen Distributionseinheit lesbare und ausführbare Programmabschnitte gespeichert sind, um alle Schritte des Verfahrens zum Verteilen eines vertraulichen Datensatzes auszuführen, wenn die Programmabschnitte von der zentralen Distributionseinheit ausgeführt werden.

Eine Verschlüsselung ist eine Funktion, die einen vertraulichen Datensatz auf einen verschlüsselten Datensatz abbildet. Insbesondere kann der vertrauliche Datensatz nicht aus dem verschlüsselten Datensatz rekonstruiert werden. Eine Entschlüsselung ist eine Funktion, die den verschlüsselten Datensatz wieder auf den vertraulichen Datensatz abbildet, in anderen Worten ist die Entschlüsselung also die inverse Funktion der Verschlüsselung. Bei einem symmetrischen Kryptographiesystem basieren die Verschlüsselung und die Entschlüsselung insbesondere auf demselben Datenschlüssel (bzw. auf einem ersten und einem zweiten Schlüssel, wobei der zweite Schlüssel aus dem ersten Schlüssel berechnet werden kann und umgekehrt. Bei einem asymmetrischen Kryptographiesystem basiert die Verschlüsselung und die Entschlüsselung insbesondere auf einem Schlüsselpaar umfassend einen öffentlichen Schlüssel und einen geheimen Schlüssel, wobei der öffentliche Schlüssel aus dem geheimen Schlüssel berechenbar ist, und wobei insbesondere der geheime Schlüssel nicht oder nur mit einer Brute-Force-Methode aus dem öffentlichen Schlüssel berechnet werden kann. Dabei basiert die Verschlüsselung insbesondere nicht auf dem geheimen Schlüssel.

Im Folgenden wird die Erfindung anhand der in den Figuren dargestellten Ausführungsbeispiele näher beschrieben und erläutert.

Fig. 1    zeigt ein Übertragungssystem
Fig. 2    zeigt ein Ablaufdiagramm eines Verfahrens zum Übertragen eines vertraulichen Datensatzes,
Fig. 3    zeigt ein erstes Ausführungsbeispiel der Datenstrukturen beim Übertragen eines vertraulichen Datensatzes,
Fig. 4    zeigt ein zweites Ausführungsbeispiel der Datenstrukturen beim Übertragen eines vertraulichen Datensatzes,
Fig. 5    zeigt ein drittes Ausführungsbeispiel der Datenstrukturen beim Übertragen eines vertraulichen Datensatzes.

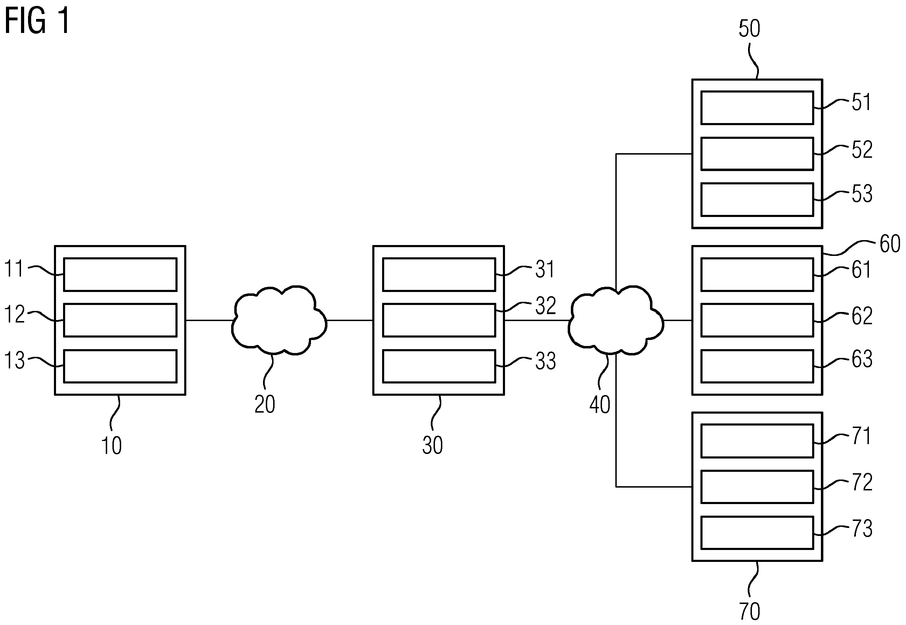

Fig. 1 zeigt ein Übertragungssystem, umfassend eine Datenquelle 10, eine zentrale Distributionseinheit 30, sowie mehrere Verarbeitungseinheiten 50, 60, 70 umfassend eine empfangende Verarbeitungseinheit 50. Die Datenquelle 10 ist über ein erstes Netzwerk 20 mit der zentralen Distributionseinheit 30. Die zentrale Distributionseinheit 30 ist über ein zweites Netzwerk 40 mit den Verarbeitungseinheiten 50, 60, 70 verbunden. Das hier dargestellte Übertragungssystem ist dazu ausgebildet, ein erfindungsgemäßes Verfahren auszuführen.

Bei der Datenquelle 10, der zentralen Distributionseinheit 30 und/oder den Verarbeitungseinheiten 50, 60, 70 kann es sich insbesondere jeweils um einen Computer, einen Mikrocontroller oder um einen integrierten Schaltkreis handeln. Alternativ kann es sich bei der Datenquelle 10, der zentralen Distributionseinheit 30 und/oder den Verarbeitungseinheiten 50, 60, 70 um einen realen oder virtuellen Verbund von Computern handeln (ein englischer Fachbegriff für einen realen Verbund ist "Cluster", ein englischer Fachbegriff für einen virtuellen Verbund ist "Cloud"). Insbesondere können die Datenquelle 10, die zentrale Distributionseinheit 30 und/oder die Verarbeitungseinheiten 50, 60, 70 auch als virtualisierte Instanzen auf einem Server betrieben werden.

Vorteilhafterweise handelt es sich im dargestellten Ausführungsbeispiel bei der Datenquelle 10 um einen Server in einer medizinischen Einrichtung, welches ein PACS (englisches Akronym für "Picture Archiving and Communication System"), RIS (englisches Akronym für "Radiology Information System") oder ein HIS (englisches Akronym für "Hospital Information System"). Weiterhin handelt es sich vorteilhafterweise beim dargestellten Ausführungsbeispiel bei der zentralen Distributionseinheit 30 um einen virtuellen Verbund von Rechnern. Weiterhin handelt es sich vorteilhafterweise beim dargestellten Ausführungsbeispiel bei den Verarbeitungseinheiten 50, 60, 70 um unabhängig betreibbare Workstations, die direkt durch Benutzereingaben von einem Radiologen gesteuert werden können. Vorteilhafterweise sind die Datenquelle 10, die zentrale Distributionseinheit 30 und die Verarbeitungseinheit 50, 60, 70 räumlich voneinander getrennt. Es ist aber auch denkbar, dass mehrere der Verarbeitungseinheiten 50, 60, 70 in einem räumlichen oder strukturellen Verbund ausgeführt sind, beispielsweise in Befundungseinrichtung mit mehreren Befundungsarbeitsplätzen. Es ist ebenfalls vorstellbar, dass die Datenquelle 10 mit einer oder mehreren der Verarbeitungseinheiten 50, 60, 70 in einem räumlichen oder strukturellen Verbund ausgeführt sind.

Im dargestellten Ausführungsbeispiel umfassen jeweils die Datenquelle 10, die zentrale Distributionseinheit 30 und die Verarbeitungseinheiten 50, 60, 70 eine Schnittstelle 11, 31, 51, 61, 71, eine Recheneinheit 12, 32, 52, 62, 72 und eine Speichereinheit 13, 33, 53, 63, 73. Bei einer Schnittstelle 11, 31, 51, 61, 71 kann es sich um eine Hardware- oder Softwareschnittstelle handeln (beispielsweise PCI-Bus, USB oder Firewire). Eine Recheneinheit 12, 32, 52, 62, 72 kann Hardware-Elemente oder Software-Elemente aufweisen, beispielsweise einen Mikroprozessor oder ein sogenanntes FPGA (englisches Akronym für "Field Programmable Gate Array"). Eine Speichereinheit 13, 33, 53, 63, 73 kann als nicht dauerhafte Arbeitsspeicher (Random Access Memory, kurz RAM) und/oder als dauerhafter Massenspeicher (Festplatte, USB-Stick, SD-Karte, Solid State Disk) realisiert sein.

Das erste Netzwerk 20 und das zweite Netzwerk 40 können identisch sein. Beim ersten Netzwerk 20 und/oder beim zweiten Netzwerk 40 kann es sich um ein lokales Netzwerk (ein englischer Fachbegriff ist "Local Area Network", kurz "LAN") oder um ein großräumiges Netzwerk (ein englischer Fachbegriff ist "Wide Area Network", kurz "WAN") handeln. Ein Beispiel für ein lokales Netzwerk ist ein Intranet, ein Beispiel für ein großräumiges Netzwerk ist das Internet. Das erste Netzwerk 20 und/oder das zweite Netzwerk 40 können insbesondere auch drahtlos ausgeführt sein, insbesondere als WLAN (für "wireless LAN", im englischen ist die Abkürzung "WiFi" gebräuchlich) oder als Bluetooth-Verbindung. Das erste Netzwerk 20 und/oder das zweite Netzwerk 40 können auch als Kombination der genannten Beispiele ausgeführt sein. Vorteilhafterweise handelt es sich bei dem hier dargestellten Ausführungsbeispiel sowohl beim ersten Netzwerk 20 als auch beim zweiten Netzwerk 40 um das Internet.

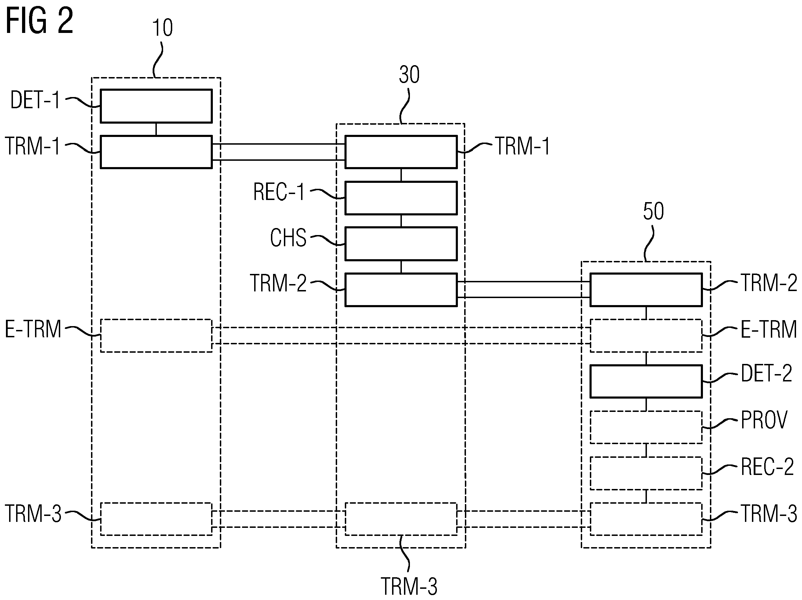

Fig. 2 zeigt ein Ablaufdiagramm eines Ausführungsbeispiels eines erfindungsgemäßen Verfahrens zum Übertragen eines vertraulichen Datensatzes 80.

Der erste Schritt des dargestellten Ausführungsbeispiels ist das erste Bestimmen DET-1 eines Transaktionsdatensatzes 82 umfassend einen verschlüsselten Datensatz 81 mittels einer Datenquelle 10, wobei der verschlüsselte Datensatz 81 auf einer Verschlüsselung des vertraulichen Datensatzes 80 basiert. Die Verschlüsselung ist hierbei eine symmetrische Verschlüsselung mittels des AES-256 (AES ist ein englisches Akronym für "Advanced Encryption Standard", eine deutsche Übersetzung ist "fortgeschrittener Verschlüsselungsstandard", die angehängte Zahl gibt die Schlüssellänge des Datenschlüssels 90 in Bit an).

Der vertrauliche Datensatz 80 ist in diesem Ausführungsbeispiel ein medizinischer Bilddatensatz (beispielsweise im DICOM-Format), für den eine Fernbefundung durchgeführt werden soll. Neben dem verschlüsselten Datensatz 81 umfasst der Transaktionsdatensatz 82 in diesem Ausführungsbeispiel noch eine Verarbeitungsinformation, die eine notwendige medizinische Qualifikation für die Befundung des vertraulichen Datensatzes 80 sowie eine angestrebte Dauer bis zum Vorliegen der Fernbefundung und einen angestrebten Preisrahmen der Fernbefundung umfasst. Die notwendige Qualifikation für die Befundung des vertraulichen Datensatzes 80 ist hierbei eine zweite Verarbeitungsbedingung.

Alternative symmetrische Verschlüsselungsverfahren sind DES (englisches Akronym für "Data Encryption Standard"), Triple-DES, IDEA (englisches Akronym für "International Data Encryption Algorithm") oder Blowfish. Es sind aber auch alle anderen symmetrischen Verschlüsselungsverfahren anwendbar, insbesondere ist das erfindungsgemäße Verfahren von der konkreten Wahl des symmetrischen Verschlüsselungsverfahrens unabhängig.

Der nächste Schritt des dargestellten Ausführungsbeispiels ist das erste Übertragen TRM-1 des Transaktionsdatensatzes 82 von der Datenquelle 10 zu einer zentralen Distributionseinheit 30 über das erste Netzwerk 20. Anschließend kann der Transaktionsdatensatz 82 insbesondere in der Speichereinheit 33 der zentralen Distributionseinheit 30 gespeichert werden.

Der nächste Schritt des dargestellten Ausführungsbeispiels ist das erste Empfangen REC-1 einer ersten Verarbeitungsbedingung durch die zentrale Distributionseinheit 30, wobei die erste Verarbeitungsbedingung eine Bedingung für die Verarbeitbarkeit des vertraulichen Datensatzes 80 durch eine von mehreren Verarbeitungseinheiten 50, 60, 70 ist.

In diesem Ausführungsbeispiel umfasst die erste Verarbeitungsbedingung sowohl die Existenz einer vertraglichen Regelung zwischen der Datenquelle 10 und den Verarbeitungseinheiten 50, 60, 70, als auch eine Information über die Verfügbarkeit der Verarbeitungseinheiten 50, 60, 70. Die Existenz der vertraglichen Regelungen wird hierbei durch eine Tabelle dargestellt, deren horizontale Dimension die mit der zentrale Distributionseinheit 30 verbundenen Datenquellen 10 entspricht, und deren vertikale Dimension den mit der zentralen Distributionseinheit 30 verbundenen Verarbeitungseinheiten 50, 60, 70 entspricht. In dieser Tabelle ist in einer Zelle, die einer Datenquelle 10 und einer der Verarbeitungseinheiten 50, 60, 70 entspricht, eine "1" oder ein "WAHR" eingetragen, wenn eine Vertragliche Regelung zwischen dem Betreiber der entsprechenden Datenquelle 10 (hier einem Krankenhaus) und dem Betreiber der entsprechenden Verarbeitungseinheit 50, 60, 70 (hier einem Radiologen) existiert, und eine "0" oder ein "FALSCH" eingetragen, wenn keine solche vertragliche Regelung existiert.

Die Information über die Verfügbarkeit der Verarbeitungseinheiten 50, 60, 70 ist eine Liste mit allen Verarbeitungseinheiten 50, 60, 70, die derzeit in Betrieb sind. Diese Liste wird in diesem Beispiel durch eine regelmäßige Anfrage an alle Verarbeitungseinheiten 50, 60, 70 erstellt und/oder aktualisiert. Alternativ können sich die einzelnen Verarbeitungseinheiten 50, 60, 70 sich jeweils bei der zentralen Distributionseinheit 30 anmelden oder abmelden.

Der nächste Schritt des dargestellten Ausführungsbeispiels ist das Auswählen CHS einer empfangenden Verarbeitungseinheit 50 aus den Verarbeitungseinheiten 50, 60, 70 mittels der zentralen Distributionseinheit 30, wobei die empfangende Verarbeitungseinheit 50 die erste Verarbeitungsbedingung erfüllt. In diesem Ausführungsbeispiel erfüllt die empfangende Verarbeitungseinheit 50 die erste Verarbeitungsbedingung, wenn zwischen dem Betreiber der Datenquelle 10 und dem Betreiber der empfangenden Verarbeitungseinheit 50 eine vertragliche Regelung existiert, und wenn die empfangende Verarbeitungseinheit 50 zum Zeitpunkt des Auswählens in Betrieb ist. Das Erfüllen der ersten Verarbeitungsbedingung kann insbesondere durch das Abfragen der beschriebenen Tabelle und der beschriebenen Liste schnell und effizient erfolgen.

Hierbei wird in diesem Ausführungsbeispiel eine Untermenge der Verarbeitungseinheiten 50, 60, 70 bestimmt, wobei jede Verarbeitungseinheit 50, 60, 70 der Untermenge der Verarbeitungseinheiten 50, 60, 70 die erste Verarbeitungsbedingung erfüllt. Die empfangende Verarbeitungseinheit 50 kann hierbei basierend auf der Verarbeitungsinformation aus der Untermenge der Verarbeitungseinheiten 50, 60, 70 ausgewählt werden.

Im dargestellten Ausführungsbeispiel wird hierzu für jede der Verarbeitungseinheiten 50, 60, 70 der Untermenge der Verarbeitungseinheiten 50, 60, 70 eine Kostenfunktion ausgewertet. Diese Kostenfunktion hat hierbei die folgende Struktur:

$$K(d, \ d_0, \ p, \ p_0) \ = \ a_d \cdot (d \ - \ d_0) \ - \ a_p \cdot (p \ - \ p_0)$$

Hierbei ist $d_0$ die angestrebte Dauer bis zum Vorliegen der Fernbefundung und $p_0$ der angestrebte Preisrahmen der Fernbefundung, welche durch die Verarbeitungsinformation gegeben sind. Der Parameter $d$ ist die voraussichtliche Dauer bis zum Vorliegen der Fernbefundung bei einer Verarbeitungseinheit 50, 60, 70, der Parameter $p$ ist der von der Verarbeitungseinheit 50, 60, 70 veranschlagte Preis für die Fernbefundung. Die Parameter $d$ und $p$ werden in diesem Ausführungsbeispiel von den Verarbeitungseinheiten 50, 60, 70 abgefragt, alternativ kann insbesondere der Parameter $d$ anhand der bereits an die Verarbeitungseinheiten 50, 60, 70 verteilten Transaktionsdatensätze 82 automatisch durch die zentrale Distributionseinheit 30 ermittelt werden. Die Parameter $a_d$ und $a_p$ sind positive Gewichtungsfaktoren, die entweder einheitlich in der zentralen Distributionseinheit 30 festgelegt wurden, oder die von der Datenquelle 10 im Transaktionsdatensatz 82 vorgegeben wurden. Im dargestellten Ausführungsbeispiel wird diejenige Verarbeitungseinheit 50, 60, 70 als empfangende Verarbeitungseinheit 50 aus der Untermenge der Verarbeitungseinheiten 50, 60, 70 ausgewählt, deren Kostenfunktion minimal ist. Weiterhin fliest in diesem Ausführungsbeispiel noch die für die Fernbefundung notwendige Qualifikation in die Kostenfunktion ein. Hierbei können die verfügbaren Qualifikationen der Verarbeitungseinheiten 50, 60, 70 ebenfalls in der zentralen Distributionseinheit 30 gespeichert sein, oder durch diese bei Bedarf abgefragt werden. Eine Qualifikation ist hierbei beispielsweise die Befähigung zur Befundung eines bestimmten Körperteils oder eines bestimmten Bilddatensatzes (Röntgenbild, Computertomographieaufnahme, Magnetresonanzaufnahme). Die Qualifikation kann sich insbesondere auf einen an der Verarbeitungseinheit 50, 60, 70 eingeloggten Anwender basieren. Erfüllt eine Verarbeitungseinheit 50, 60, 70 nicht die notwendige Qualifikation, wird in diesem Ausführungsbeispiel die Kostenfunktion auf einen Maximalwert gesetzt.

Alternativ kann das Auswählen CHS der empfangenden Verarbeitungseinheit 50 auch zufällig aus der Untermenge der Verarbeitungseinheiten 50, 60, 70 oder aus den Verarbeitungseinheiten 50, 60, 70 erfolgen, insbesondere so, dass jede der Verarbeitungseinheiten 50, 60, 70 die gleiche Auswahlwahrscheinlichkeit aufweist.

Der nächste Schritt des dargestellten Ausführungsbeispiels ist das zweite Übertragen TRM-2 des Transaktionsdatensatzes 82 von der zentralen Distributionseinheit 30 zu der empfangenden Verarbeitungseinheit 50, hier über das zweite Netzwerk 40. Anschließend wird der Transaktionsdatensatz 82 in der Speichereinheit 53 der empfangenden Verarbeitungseinheit 50 gespeichert. Im dargestellten Ausführungsbeispiel liegt der Datenschlüssel 90 zu diesem Zeitpunkt noch nicht bei der empfangenden Verarbeitungseinheit 50 vor, dadurch kann noch kein Zugriff auf den vertraulichen Datensatz 80 erfolgen. Hierdurch ist es möglich, den Transaktionsdatensatz 82 zurückzurufen, in anderen Worten den Datenschlüssel 90 nie zu übertragen. Alternativ kann der Datenschlüssel 90 auch vorab bei der empfangenden Verarbeitungseinheit 50 vorliegen, in diesem Fall kann der vertrauliche Datensatz zumindest theoretisch direkt nach dem zweiten Übertragen TRM-2 bestimmt werden.

Der nächste Schritt des dargestellten Ausführungsbeispiels ist das verschlüsseltes Übertragen E-TRM des Datenschlüssels 90 von der Datenquelle 10 zu der empfangenden Verarbeitungseinheit 50. Dieser Schritt wird in diesem Ausführungsbeispiel als Reaktion auf den erstmaligen Versuch der empfangenden Verarbeitungseinheit 50 ausgeführt, den vertraulichen Datensatz 80 zum Zweck der Befundung zu öffnen. Insbesondere wird hierbei eine Anforderungsnachricht von der empfangenden Verarbeitungseinheit 50 über das zweite Netzwerk 40, die zentrale Distributionseinheit 30 und das erste Netzwerk 20 an die Datenquelle 10 geschickt, die als Reaktion den Datenschlüssel 90 verschlüsselt über das erste Netzwerk 20, die zentrale Distributionseinheit 30 und das zweite Netzwerk 40 an die empfangende Verarbeitungseinheit 50 überträgt.

Der Schritt des verschlüsselten Übertragen E-TRM des Datenschlüssels 90 von der Datenquelle 10 zu der empfangenden Verarbeitungseinheit 50 ist optional, wenn der Datenschlüssel 90 der empfangenden Verarbeitungseinheit 50 bereits bekannt ist.

Das verschlüsselte Übertragen E-TRM basiert in diesem Ausführungsbeispiel auf dem asymmetrischen Verschlüsselungsverfahren RSA. Hierbei ist jedem der Verarbeitungseinheiten 50, 60, 70 ein Schlüsselpaar umfassend einen öffentlichen Schlüssel 91 und einen geheimen Schlüssel 92 zugeordnet. Hierbei ist der öffentliche Schlüssel der Datenquelle 10 bekannt, der geheime Schlüssel 92 ist nur der zugehörigen Verarbeitungseinheit 50, 60, 70 bekannt. Die Datenquelle 10 kann mittels des öffentlichen Schlüssels 91 den Datenschlüssel 90 in einen verschlüsselten Datenschlüssel 83 umwandeln, und die empfangende Verarbeitungseinheit 50 kann mittels des geheimen Schlüssels 92 den verschlüsselten Datenschlüssel 83 in den ursprünglichen Datenschlüssel 90 umwandeln.

Es wäre prinzipiell denkbar, zunächst nur die erste Verarbeitungsbedingung und die optionale Verarbeitungsinformation von der Datenquelle 10 an die zentrale Distributionseinheit 30 zu übermitteln, und erst nach dem Auswählen CHS der empfangenden Verarbeitungseinheit 50 (also zu einem Zeitpunkt, zu dem die empfangende Verarbeitungseinheit 50 und somit auch der zugehörige öffentliche Schlüssel 91 bekannt ist) den verschlüsselten Datensatz 81 mittels einer asymmetrischen Verschlüsse aus dem vertraulichen Datensatz 80 zu generieren. Zum einen ist eine asymmetrische Verschlüsselung des vertraulichen Datensatzes 80 (der durchaus mehrere Gigabyte Daten umfassen kann) deutlich langsamer als eine symmetrische Verschlüsselung, auf der anderen Seite kann der verschlüsselte Datensatz 81 dann erst auf Aufforderung der empfangenden Verarbeitungseinheit 50 geschickt werden, also normalerweise zu einem Zeitpunkt, an dem der Anwender mit dem Befunden beginnen möchte. Aufgrund des großen Datenvolumens würde sich eine deutlich zu große Latenz einstellen, bis mit der eigentlichen Befundung begonnen werden kann. Eine Übertragung vorab würde in diesem alternativen Verfahren bedeuten, dass die Übertragung des Transaktionsdatensatzes 82 nicht rückgängig gemacht werden kann.

Der nächste Schritt des dargestellten Ausführungsbeispiels ist das zweite Bestimmen DET-2 des vertraulichen Datensatzes 80 basierend auf einer Entschlüsselung des verschlüsselten Datensatzes 81 mittels der empfangenden Verarbeitungseinheit 50.

Die nächsten Schritte des dargestellten Ausführungsbeispiels sind das Bereitstellen PROV des vertraulichen Datensatzes 80 zur medizinischen Befundung mittels der empfangenden Verarbeitungseinheit 50, das zweite Empfangen REC-2 einer Befundungsinformation mittels der empfangenden Verarbeitungseinheit 50, und das dritte Übertragen TRM-3 der Befundungsinformation von der empfangenden Verarbeitungseinheit 50 zur Datenquelle 10. Diese Schritte sind optional, alternativ kann in anderen Ausführungsbeispielen auch keine Rückmeldung zur Datenquelle 10 erfolgen, wiederum alternativ kann in anderen Ausführungsbeispielen die Rückmeldung bzw. die Übermittlung der Befundungsinformation auch über einen anderen Kommunikationskanal erfolgen, beispielsweise telefonisch.

Das Bereitstellen PROV des vertraulichen Datensatzes 80 erfolgt hierbei insbesondere durch das Anzeigen des vertraulichen Datensatzes 80 auf einem Ausgabegerät eines Anwenders der empfangenden Verarbeitungseinheit 50. Hierbei können dem Anwender vorteilhafterweise Eingabemittel bereitgestellt werden, die eine Manipulation der Anzeige des vertraulichen Datensatzes 80 ermöglichen. Ist der vertrauliche Datensatz 80 ein Bilddatensatz, können beispielsweise eine Projektionsrichtung- und Projektionsart empfangen werden, eine Helligkeits- und Kontrasteinstellung und/oder eine Festlegung eines Bildausschnittes.

Das zweite Empfangen REC-2 der Befundungsinformation kann über die Schnittstelle 53 der empfangenden Verarbeitungseinheit 50 erfolgen, alternativ mittels einer Eingabeeinheit der empfangenden Verarbeitungseinheit 50 (beispielsweise eine Tastatur oder ein Mikrophon). Bei der Befundungsinformation kann es sich insbesondere um unstrukturierte Information (z.B. in Form eines Freitextes) oder um strukturierte Information (z.B. in Form einer XML-Datei) handeln.

DAs dritte Übertragen TRM-3 der Befundungsinformation von der empfangenden Verarbeitungseinheit 50 zur Datenquelle 10 kann insbesondere verschlüsselt erfolgen. Hierbei kann wiederum ein asymmetrisches Kryptographiesystem verwendet werden, mit dem Unterschied, dass die Datenquelle 10 als Empfänger der Befundungsinformation bereits bekannt ist, und damit auch der öffentliche Schlüssel der Datenquelle 10. Weiterhin kann das dritte Übertragen TRM-3 insbesondere über die zentrale Distributionseinheit 30 erfolgen. Die Befundungsinformation kann dann durch die Datenquelle 10 weiterverarbeitet, dargestellt oder zusammen mit dem vertraulichen Datensatz 80 gespeichert werden.

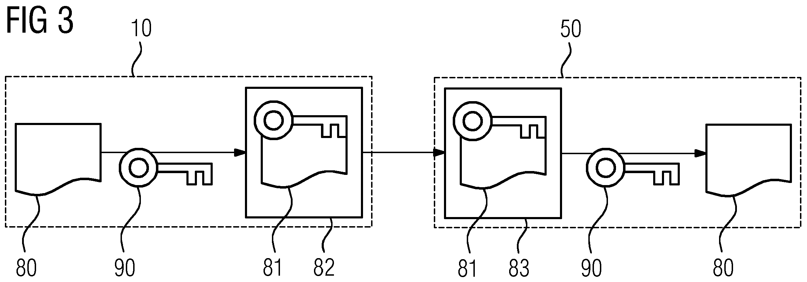

Fig. 3, Fig. 4 und Fig. 5 zeigen ein Ausführungsbeispiele der Datenstruktur beim Verfahren zum Übertragen eines vertraulichen Datensatzes 80, insbesondere bei der Datenquelle 10 und der empfangenden Verarbeitungseinheit 50. Auf die Darstellung der zentralen Distributionseinheit 30 wurde jeweils verzichtet, da dieses vorteilhafterweise den Transaktionsdatensatz 82 nicht verändert.

In Fig. 3 umfasst der Transaktionsdatensatz 82 den verschlüsselten Datensatz 81, wobei der verschlüsselte Datensatz 81 durch eine symmetrische Verschlüsselung mit dem Datenschlüssel 90 aus dem vertraulichen Datensatz 80 hervorgeht. Die empfangende Verarbeitungseinheit 50 kann den vertraulichen Datensatz 80 aus dem Transaktionsdatensatz 82 rekonstruieren, indem der im Transaktionsdatensatz 82 enthaltene verschlüsselte Datensatz 81 mit dem Datenschlüssel 90 symmetrisch entschlüsselt wird. Hierbei muss der Datenschlüssel 90 sowohl der Datenquelle 10 und der empfangenden Verarbeitungseinheit 50 bekannt sein. Dies kann beispielsweise dadurch erreicht werden, dass der Datenschlüssel 90 bereits bei der Produktion bzw. Erstellung der Datenquelle 10 und der empfangenden Verarbeitungseinheit 50 in die Datenquelle 10 und die empfangende Verarbeitungseinheit 50 integriert wird. Alternativ kann der Datenschlüssel 90 auch über einen sicheren Kommunikationskanal übertragen werden, beispielsweise ein VPN (englisches Akronym für "virtual private network").

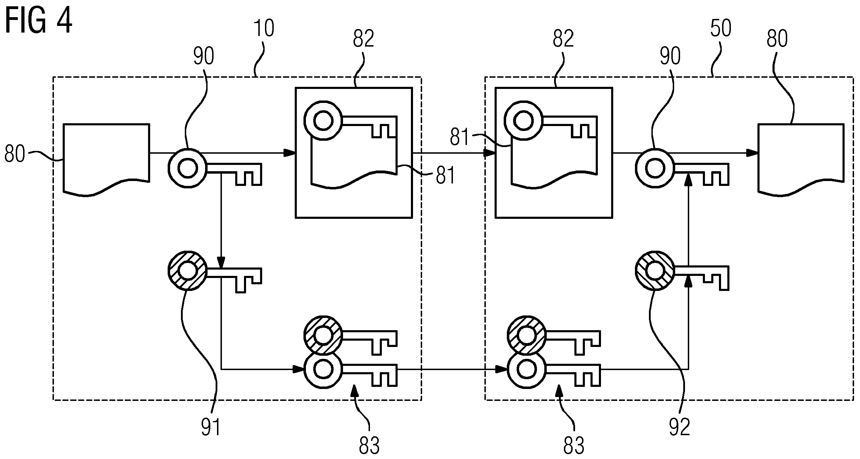

In Fig. 4 ist zusätzlich zur Fig. 3 eine Methode dargestellt, den Datenschlüssel 90 von der Datenquelle 10 zur empfangenden Verarbeitungseinheit 50 zu übertragen. Diese Methode basiert auf einem asymmetrischen Kryptographiesystem, hier dem RSA-Verfahren (Akronym für die Erfinder Rivest, Shamir und Adleman). Alternativ können auch andere System verwendet werden, beispielsweise Diffie-Hellman, Merkle-Hellmann oder auf elliptischen Kurven basierende System. Hierfür ist der empfangenden Verarbeitungseinheit 50 ein Schlüsselpaar, umfassend einen öffentlichen Schlüssel 91 und einen geheimen Schlüssel 92, zugeordnet, wobei der öffentliche Schlüssel 91 öffentlich und damit auch der Datenquelle 10 bekannt ist, und der geheime Schlüssel 92 nur der empfangenden Verarbeitungseinheit 50 bekannt ist.

Durch Anwendung der auf dem öffentlichen Schlüssel 91 basierenden Verschlüsselungsfunktion des asymmetrischen Kryptographiesystems auf den Datenschlüssel 90 wird durch die Datenquelle 10 ein verschlüsselter Datenschlüssel 83 erzeugt. Dieser verschlüsselte Datenschlüssel 83 kann direkt oder über die zentrale Distributionseinheit 30 zur empfangenden Verarbeitungseinheit 50 übertragen werden. Die empfangende Verarbeitungseinheit 50 kann dann durch Anwendung der auf dem geheimen Schlüssel 92 basierenden Entschlüsselungsfunktion des asymmetrischen Kryptographiesystems auf den verschlüsselten Datenschlüssel 83 den Datenschlüssel 90 rekonstruieren, und anschließend den Datenschlüssel 90 zur Bestimmung des vertraulichen Datensatzes 80 verwenden. Die Verschlüsselungsfunktion des asymmetrischen Kryptographiesystems und die Entschlüsselungsfunktion des asymmetrischen Kryptographiesystems können hierbei identisch oder verschieden sein.

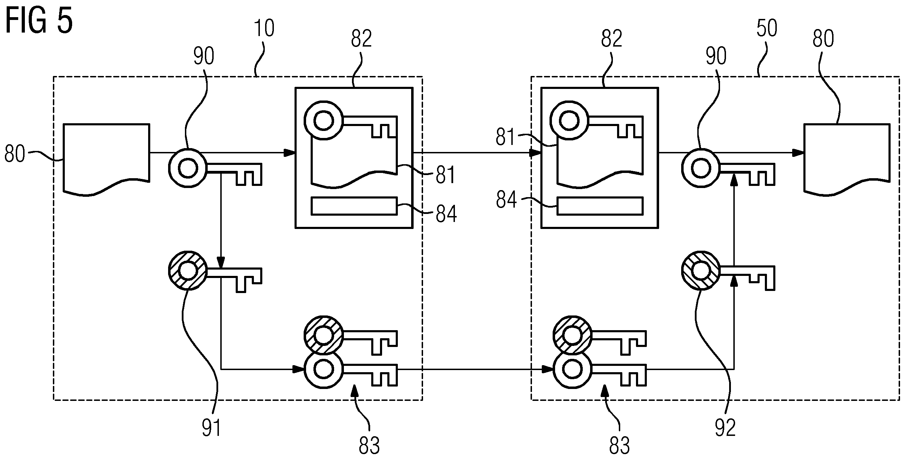

In Fig. 5 umfasst der Transaktionsdatensatz 82 neben dem verschlüsselten Datensatz 81 weiterhin eine Signatur 84 des verschlüsselten Datensatzes 81. Hierzu muss im Rahmen eines asymmetrischen Kryptographiesystems auch der Datenquelle 10 ein geheimer und ein öffentlicher Schlüssel zugeordnet sein. Das asymmetrische Kryptographiesystem zur Bestimmung der Signatur 84 kann hierbei auch vom asymmetrischen Kryptographiesystem zur verschlüsselten Übertragung E-TRM des Datenschlüssels 90 verschieden sein, vorteilhafterweise sind beide Kryptographiesysteme aber identisch. Zur Bestimmung der Signatur 84 wird dann mittels des geheimen Schlüssels der Datenquelle 10 eine Signaturfunktion auf den verschlüsselten Datensatz 81 oder den vertraulichen Datensatz 80 angewendet. Alternativ kann die Signaturfunktion auch auf einen Hashwert des verschlüsselten Datensatzes 81 oder es vertraulichen Datensatzes 80 angewendet werden. Zur Verifizierung der Signatur 84 wird dann auf der empfangenden Verarbeitungseinheit 50 mittels des öffentlichen Schlüssels der Datenquelle 10 eine Verifikationsfunktion auf die Signatur angewendet, und mit dem verschlüsselten Datensatz 81, dem vertraulichen Datensatz 80, dem Hash des verschlüsselten Datensatzes 81 oder dem Hash des vertraulichen Datensatzes 80 verglichen.

## Patentansprüche

1. Verfahren zum Übertragen eines vertraulichen Datensatzes (80), umfassend die folgenden Verfahrensschritte:

    - Erstes Bestimmen (DET-1) eines Transaktionsdatensatzes (82) umfassend einen verschlüsselten Datensatz

(81) mittels einer Datenquelle (10),
wobei der verschlüsselte Datensatz (81) auf einer Verschlüsselung des vertraulichen Datensatzes (80) basiert,
- Erstes Übertragen (TRM-1) des Transaktionsdatensatzes (82) von der Datenquelle (10) zu einer zentralen Distributionseinheit (30),
- Erstes Empfangen (REC-1) einer ersten Verarbeitungsbedingung durch die zentrale Distributionseinheit (30), wobei die erste Verarbeitungsbedingung eine Bedingung für die Verarbeitbarkeit des vertraulichen Datensatzes (80) durch eine von mehreren Verarbeitungseinheiten (50, 60, 70) ist,
- Auswählen (CHS) einer empfangenden Verarbeitungseinheit (50) aus den Verarbeitungseinheiten (50, 60, 70) mittels der zentralen Distributionseinheit (30),
wobei die empfangende Verarbeitungseinheit (50) die erste Verarbeitungsbedingung erfüllt,
- Zweites Übertragen (TRM-2) des Transaktionsdatensatzes (82) von der zentralen Distributionseinheit (30) zu der empfangenden Verarbeitungseinheit (50),
- Zweites Bestimmen (DET-2) des vertraulichen Datensatzes (80) basierend auf einer Entschlüsselung des verschlüsselten Datensatzes (81) mittels der empfangenden Verarbeitungseinheit (50).

2. Verfahren nach dem Anspruch 1, wobei der vertrauliche Datensatz (80) ein medizinischer Datensatz, insbesondere ein medizinischer Bilddatensatz ist, weiterhin umfassend:

- Bereitstellen (PROV) des vertraulichen Datensatzes (80) zur medizinischen Befundung mittels der empfangenden Verarbeitungseinheit (50),
- Zweites Empfangen (REC-2) einer Befundungsinformation mittels der empfangenden Verarbeitungseinheit (50),
- Drittes Übertragen (TRM-3) der Befundungsinformation von der empfangenden Verarbeitungseinheit (50) zur Datenquelle (10) .

3. Verfahren nach dem Anspruch 1 oder 2, wobei die Verschlüsselung des vertraulichen Datensatzes (80) eine symmetrische Verschlüsselung basierend auf einem Datenschlüssel (90) ist, wobei die Entschlüsselung des verschlüsselten Datensatzes (81) eine symmetrische Entschlüsselung basierend auf dem Datenschlüssel (90) ist, weiterhin umfassend den folgenden Verfahrensschritt:

- Verschlüsseltes Übertragen (E-TRM) des Datenschlüssels (90) von der Datenquelle (10) zu der empfangenden Verarbeitungseinheit (50).

4. Verfahren nach dem Anspruch 3, wobei der Datenschlüssel (90) nur für die Verschlüsselung von genau einem vertraulichen Datensatz (80) verwendet wird.

5. Verfahren nach dem Anspruch 3 oder 4, wobei das verschlüsselte Übertragen (E-TRM) des Datenschlüssels (90) auf einem asymmetrischen Kryptographiesystem basiert.

6. Verfahren nach einem der Ansprüche 3 bis 5, wobei das verschlüsselte Übertragen (E-TRM) von der empfangenden Verarbeitungseinheit (50) initiiert wird, wenn mittels der empfangenden Verarbeitungseinheit (50) erstmals ein Zugriff auf den vertraulichen Datensatz (80) erfolgt.

7. Verfahren nach einem der vorstehenden Ansprüche, wobei die erste Verarbeitungsbedingung darauf basiert, ob eine vertragliche Regelung zwischen einem ersten Betreiber der Datenquelle (10) und einem zweiten Betreiber einer der Verarbeitungseinheiten (50, 60, 70) besteht.

8. Verfahren nach einem der Ansprüche 2 bis 7, wobei beim ersten Empfangen (REC-1) weiterhin eine Verarbeitungsinformation empfangen wird,
wobei die Verarbeitungsinformation wenigstens einen der folgenden Parameter betrifft:

- Zeitraum, der zur Befundung des vertraulichen Datensatzes (80) zur Verfügung steht,
- Notwendige Qualifikation zur Befundung des vertraulichen Datensatzes (80),
- Preisrahmen für eine Befundung des vertraulichen Datensatzes (80).

9. Verfahren nach dem Anspruch 8, wobei die Verarbeitungsinformation eine zweite Verarbeitungsbedingung umfasst, und wobei die empfangende Verarbeitungseinheit (50) die zweite Verarbeitungsbedingung erfüllt.

10. Verfahren nach dem Anspruch 9, wobei die empfangende Verarbeitungseinheit (50) zufällig aus einer Untermenge der Verarbeitungseinheiten (50, 60, 70) ausgewählt wird, wobei jede der Verarbeitungseinheiten (50, 60, 70) der Untermenge die erste Verarbeitungsbedingung und die zweite Verarbeitungsbedingung erfüllt.

11. Verfahren nach einem der Ansprüche 8 oder 9, wobei das Auswählen (CHS) der empfangenden Verarbeitungseinheit (50) aus den Verarbeitungseinheiten (50, 60, 70) auf der Auswertung einer Kostenfunktion für die Verarbeitungseinheiten (50, 60, 70) basiert, und wobei die Kostenfunktion auf der Verarbeitungsinformation basiert.

12. Übertragungssystem zum Übertragen eines vertraulichen Datensatzes (80), umfassend die folgenden Einheiten:

- Datenquelle (10), ausgebildet zum ersten Bestimmen (DET-1) eines Transaktionsdatensatzes (82) umfassend einen verschlüsselten Datensatz (81), wobei der verschlüsselte Datensatz (81) auf einer Verschlüsselung des vertraulichen Datensatzes (80) basiert, weiterhin ausgebildet zum ersten Übertragen (TRM-1) des Transaktionsdatensatzes (82) zu einer zentralen Distributionseinheit (30),
- zentrale Distributionseinheit (30), ausgebildet zum ersten Empfangen (REC-1) einer ersten Verarbeitungsbedingung, wobei die erste Verarbeitungsbedingung eine Bedingung für die Verarbeitbarkeit des vertraulichen Datensatzes (80) durch eine von mehreren Verarbeitungseinheiten (50, 60, 70) ist, weiterhin ausgebildet zum Auswählen (CHS) einer empfangenden Verarbeitungseinheit (50) aus den Verarbeitungseinheiten (50, 60, 70), wobei die empfangende Verarbeitungseinheit (50) die erste Verarbeitungsbedingung erfüllt, weiterhin ausgebildet zum zweiten Übertragen (TRM-2) des Transaktionsdatensatzes (82) zu der empfangenden Verarbeitungseinheit (50),
- empfangende Verarbeitungseinheit (50), ausgebildet zum zweiten Bestimmen (DET-2) des vertraulichen Datensatzes (80) basierend auf einer Entschlüsselung des verschlüsselten Datensatzes (81).

13. Übertragungssystem nach dem Anspruch 12, weiterhin ausgebildet ein Verfahren nach einem der Ansprüche 1 bis 11 auszuführen.

14. Computerprogrammprodukt mit einem Computerprogramm, welches direkt in einen Speicher (13, 33, 53, 63, 73) eines Übertragungssystems ladbar ist, mit Programmabschnitten, um alle Schritte des Verfahrens nach einem der Ansprüche 1 bis 11 auszuführen, wenn die Programmabschnitte von dem Übertragungssystem ausgeführt werden.

15. Computerlesbares Speichermedium, auf welchem von einem Übertragungssystem lesbare und ausführbare Programmabschnitte gespeichert sind, um alle Schritte des Verfahrens nach einem der Ansprüche 1 bis 11 auszuführen, wenn die Programmabschnitte von dem Übertragungssystem ausgeführt werden.

FIG 1

EP 3 477 903 A1

# FIG 2

EP 3 477 903 A1

FIG 3

FIG 4

EP 3 477 903 A1

FIG 5

**Europäisches Patentamt**
**European Patent Office**
**Office européen des brevets**

# EUROPÄISCHER RECHERCHENBERICHT

Nummer der Anmeldung

EP 17 19 9022

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2011/015941 A1 (BACKHAUS BRENT [US]) 20. Januar 2011 (2011-01-20) * Zusammenfassung; Abbildungen 1, 2A, 4, 8, 9, 10 * * Absätze [0031], [0036], [0040] * * Absätze [0145] - [0150] * * Absatz [0171] * ----- | 1-15 | INV. H04L29/06 G06F21/62 G16H10/60 |
| X | US 2006/195339 A1 (BACKHAUS BRENT [US] ET AL) 31. August 2006 (2006-08-31) * Zusammenfassung; Abbildungen 1, 3, 4 * * Absätze [0034] - [0051] * * Absätze [0067] - [0075] * ----- | 1-15 | |
| X | US 2009/193267 A1 (CHUNG CHIASEN [CA]) 30. Juli 2009 (2009-07-30) * Zusammenfassung; Abbildungen 1, 2, 3, 4, 6 * * Absätze [0019], [0020], [0029] * * Absätze [0036] - [0039] * * Absätze [0043] - [0048] * ----- | 1-15 | |
| | | | **RECHERCHIERTE SACHGEBIETE (IPC)** H04L G06F G16H |

Der vorliegende Recherchenbericht wurde für alle Patentansprüche erstellt

| Recherchenort | Abschlußdatum der Recherche | Prüfer |
|---|---|---|
| München | 18. April 2018 | Schossmaier, Klaus |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 17 19 9022

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-04-2018

| Im Recherchenbericht angeführtes Patentdokument | Datum der Veröffentlichung | Mitglied(er) der Patentfamilie | Datum der Veröffentlichung |
|---|---|---|---|
| US 2011015941 A1 | 20-01-2011 | US 2011015941 A1<br>US 2012323593 A1<br>US 2014088987 A1 | 20-01-2011<br>20-12-2012<br>27-03-2014 |
| US 2006195339 A1 | 31-08-2006 | US 2006195339 A1<br>US 2010256986 A1<br>US 2011004490 A1<br>US 2011191118 A1<br>US 2012245949 A1<br>US 2014142969 A1 | 31-08-2006<br>07-10-2010<br>06-01-2011<br>04-08-2011<br>27-09-2012<br>22-05-2014 |
| US 2009193267 A1 | 30-07-2009 | CA 2728435 A1<br>CA 2747883 A1<br>US 2009193267 A1<br>WO 2009094770 A1 | 06-08-2009<br>06-08-2009<br>30-07-2009<br>06-08-2009 |

EPO FORM P0461

Für nähere Einzelheiten zu diesem Anhang : siehe Amtsblatt des Europäischen Patentamts, Nr.12/82